# EUROPÄISCHE PATENTSCHRIFT

(11) **EP 3 511 301 B1**
(45) Veröffentlichungstag und Bekanntmachung des Hinweises auf die Patenterteilung: **26.08.2020**
(21) Anmeldenummer: 19150725.0
(22) Anmeldetag: 08.01.2019
(51) Int. Cl.: C03B 33/02, C03B 33/06, B23K 26/02, B23K 26/53

(54) **VERFAHREN UND VORRICHTUNG ZUR HERSTELLUNG VON GLASVORPRODUKTEN UND VON GLASPRODUKTEN**
METHOD AND DEVICE FOR PRODUCING GLASS PRELIMINARY PRODUCTS AND GLASS PRODUCTS
PROCÉDÉ ET DISPOSITIF DE FABRICATION DE PRODUITS SEMI-FINIS EN VERRE ET DE PRODUITS EN VERRE

(30) Priorität: 10.01.2018 DE 102018100443
(43) Veröffentlichungstag der Anmeldung: 17.07.2019
(73) Patentinhaber: SCHOTT AG, 55122 Mainz (DE)
(72) Erfinder: WAGNER, Fabian, 55118 Mainz (DE); KLUGE, Michael, 63073 Offenbach am Main (DE); ORTNER, Andreas, 55435 Gau-Algesheim (DE); BRÜCKBAUER, Laura, 67585 Dorn-Dürkheim (DE); LENTES, Frank-Thomas, 55411 Bingen (DE)
(74) Vertreter: Blumbach · Zinngrebe Patentanwälte PartG mbB

(56) Entgegenhaltungen:
- WO-A1-2017/073118

## Beschreibung

### Gebiet der Erfindung

Die Erfindung bezieht sich auf ein Verfahren zur Herstellung von Glasvorprodukten und von Glasprodukten, auf eine Vorrichtung zur Herstellung von Glasvorprodukten oder von Glasprodukten, auf rohrförmiges Glasvorprodukt oder Glasprodukt, auf Glasrohrabschnitte, oder Glasvorproduktabschnitte, oder Glasproduktabschnitte sowie deren Verwendung für Pharmaverpackungen.

Die Erfindung bezieht sich insbesondere auf ein Verfahren zum Trennen von Rohrglas in abgemessene Glasrohrabschnitte, auf eine Vorrichtung zur Vorbereitung der Trennung von Rohrglas in abgemessene Glasrohrabschnitte sowie nach dem Verfahren hergestellte Glasrohrabschnitte und deren Verwendung. Unter Rohrglas oder Glasvorprodukt werden auch teilweise unrunde, längliche Hohlkörper und solche mit variablem Durchmesser entlang ihrer Längserstreckung verstanden.

### Hintergrund der Erfindung

Um Glasrohrabschnitte von Rohrglas abzutrennen, ist es bekannt, das Rohrglas mechanisch zu ritzen und danach zu brechen. Das Brechen kann auch durch Thermoschock erfolgen, indem ein warmes Glasrohr, im kontinuierlichen Bandzug aus der Schmelze gewonnen, mit einem kühlen Werkzeug berührt wird und dadurch in einem kleinen Bereich der Mantelfläche des Rohres ein Defekt eingebracht wird, der die Bruchebene bestimmt.

Nachteil dieser bekannten Verfahren sind die Ungenauigkeit der Länge der abgetrennten Glasrohrabschnitte sowie mangelnde Qualität der Bruchflächen an den Rohrenden. Dort entstehen Splitter beim Brechen der Rohre und die Rohrenden weisen eine verringerte Festigkeit auf. Deshalb wird typischerweise eine Nachverarbeitung der Rohrenden vorgenommen und beim Brechen entstehende Splitter oder Partikel werden durch Waschprozesse entfernt. Dies verteuert die Herstellung von rohrförmigen Glasvorprodukten, wie sie für die Herstellung von Glasprodukten für die Pharmaindustrie benötigt werden.

Aus US 2015/0034613 A1 ist die Bildung von kontinuierlichen Laserfilamenten in transparenten Materialien bekannt. Es wird ein Bündel ("burst") von ultraschnellen Laserpulsen auf das transparente Material fokussiert, das plattenförmig ist.

US 2015/0140241 A1 offenbart die Erzeugung eines schraubenförmig geschnittenen, transparenten Rohres, wobei durch das transparente Rohr ein kontinuierliches Laserfilament erzeugt wird. Von dem Rohrende können einzelne Ringe abgetrennt werden oder es wird eine schraubenförmige Glasspirale abgetrennt. Das Verfahren ist sehr energieaufwendig.

WO 2016/007843 A1 betrifft das Schneiden von Artikeln aus Glas durch Induzieren von gepulsten Laserperforationen in das Glas. Gezeigt werden u.a. ein sich relativ zu einer gepulsten Laseranordnung drehendes zylindrisches Rohr, in das radiale Laserperforationen um den Umfang des Glasrohres geschnitten werden, die sich als Defekte, Aussparungen oder Löcher im Glas darstellen. Solche genauen, engen Bohrungen machen die nachfolgende Trennung um die Perforationslinie einfacher. Es ist auch möglich, einen drehbaren Arm der gepulsten Laseranordnung um den Glasartikel kreisen zu lassen und dabei das umkreiste Rohr abzuschneiden.

WO 2017/060252 A1 betrifft ein Verfahren zur Erzeugung eines Werkstücks aus einem dielektrischen Material, welches mindestens eine Zone mit definiert eingestellter Festigkeit aufweist, wobei die Zone definierter Festigkeit Hohlräume aufweist. Die WO 2017/060252 A1 betrifft ferner ein nach dem Verfahren hergestelltes Werkstück. Die Hohlräume in dem Werkstück werden mit einem gepulsten Laser erzeugt, dessen Strahlung gemäß eines paraxialen Fokus einer Linse geformt wird. Es werden kanalförmige Hohlräume erzeugt, die das Werkstück teilweise oder vollständig durchdringen und die orthogonal zur Werkstückoberfläche ausgerichtet sind.

Die US 2018/0215649 A1 betrifft ein Schneideverfahren für ein Röhrenglas, umfassend einen Erwärmungsschritt zum Erwärmen eines voreingestellten Schnittabschnitts des Röhrenglases durch Abstrahlen von Laserlicht auf den voreingestellten Schnittabschnitt, einen Schritt zum Bilden eines inneren Rissbereichs mit einem oder mehreren Rissen durch Mehrphotonenabsorption, die in einem Bestrahlungsbereich von Laserlicht durch Abstrahlen des Laserlichts mit einem eingestellten Brennpunkt auftritt zu einer Innenseite des voreingestellten Schnittabschnitts und einen Kühlschritt zum Kühlen des voreingestellten Schnittabschnitts, um dadurch zu bewirken, dass sich die Risse im Inneren des voreingestellten Schnittabschnitts ausbreiten.

### Generelle Beschreibung der Erfindung

Der Erfindung liegt der Aufgabe zugrunde, ein Verfahren und eine Vorrichtung zur Herstellung von Glasprodukten aus rohrförmigen Glasvorprodukten anzugeben, sowie rohrförmige Glasvorprodukte oder Glasprodukte zur Verfügung zu stellen.

Ferner liegt der Erfindung die Aufgabe zugrunde, Rohrglas oder Glasvorprodukt in abgemessene Abschnitte durch Brechen zu trennen, ohne dass Splitter oder Partikel an der Bruchfläche entstehen. Damit sollen Waschprozesse und ggf. eine Rohrendnachbearbeitung vermieden werden, und zwar ohne Inkaufnahme eines energiereichen, Laser-basierten Abtrennungsprozesses.

Als Rohrglas kann Pharmarohr bis 50 mm Durchmesser, oder technisches Rohr, z.B. aus Duran-Glas, verwendet werden.

Als Glasvorprodukt ist umgeformtes Rohrglas anzusehen, auch in Rohrglasabschnitte aufgeteilt. Als Glasprodukt sollen Ampullen, Karpulen oder Spritzenkörper hergestellt werden können. Diese Produkte sollen mit Sollbruchstellen zum späteren Abtrennen versehen werden können.

Die gestellte Aufgabe wird durch den Gegenstand der unabhängigen Ansprüche gelöst. Die abhängigen Ansprüche beinhalten Ausgestaltungen und Weiterentwicklungen der Erfindung. Gemäß der Erfindung wird Rohrglas oder rohrförmiges Glasvorprodukt entlang von Sollbruchebenen mit schräg zur örtlichen Radialen verlaufenden Filamenten versehen, die das saubere Trennen des Rohrglases oder des Glasvorproduktes, oder eines Glasendproduktes ermöglichen. Als Filamente werden im Sinne der Erfindung linien- oder filamentförmige Schädigungen im Glas verstanden, die von außen nach innen im Glaskörper verlaufen.

Das Verfahren zur Herstellung von Glasvorprodukten und von Glasprodukten beinhaltet ein Verfahren zum Trennen von Rohrglas in abgemessene Glasrohrabschnitte, mit folgenden Schritten:
- Bereitstellen von translatorisch stehendem oder translatorisch bewegtem Glasrohr
- Laser-basiertes Bestrahlen des Glasrohres mit fokussierter Strahlung zur Erzeugung von Filamenten in einer gewünschten Trennebene des Glasrohres, wobei
- die Bestrahlung mit fokussierter Strahlung in der Trennebene zur Erzeugung der Filamente in einem von der Senkrechten abweichenden Bestrahlungsschrägwinkel (α) zur örtlichen Oberfläche des Glasrohres erfolgt, wobei die Filamente von außen nach innen verlaufen,
- Abtrennen des abgemessenen Glasrohrabschnittes durch mechanisch oder thermisch eingebrachte (Zug-)Spannung in der Trennebene des Glasrohrs und Erzeugung einer Bruchfläche entlang der Trennebene.

Insbesondere arbeitet das erfindungsgemäße Verfahren zum Trennen von Rohrglas in abgemessene Glasrohrabschnitte mit folgenden Schritten:
a) Bereitstellen von Glasrohr,
b) Laser-basiertes Bestrahlen des Glasrohrs mit fokussierter Strahlung in einem Bestrahlungsschrägwinkel zur Erzeugung von Filamenten in einer gewünschten Trennebene des Glasrohres, wobei die Bestrahlung mit fokussierter Strahlung in der Trennebene zur Erzeugung der Filamente in einem von der Senkrechten abweichenden Bestrahlungswinkel zur örtlichen Oberfläche des Glasrohres erfolgt, wobei die Filamente von außen nach innen verlaufen, und
c) Abtrennen des abgemessenen Glasrohrabschnittes durch mechanisch oder thermisch eingebrachte Spannung in der Trennebene des Glasrohres und Erzeugung einer Bruchfläche entlang der Trennebene.

Das Glasrohr oder das rohrförmige Glasvorprodukt kann translatorisch stehend oder in Richtung der Längsachse translatorisch bewegt in Schritt a) bereit gestellt werden. Bei einer translatorisch stehenden Bereitstellung kann das Glasrohr oder das rohrförmige Glasvorprodukt auch um seine Längsachse während der Bearbeitung rotieren. Danach erfolgt eine schubweise translatorische Bewegung des Glasrohres oder Glasvorproduktes.

Die Besonderheit des erfindungsgemäßen Verfahrens besteht darin, dass in Schritt b) die Bestrahlung in der Trennebene zur Erzeugung der Filamente in einem Bestrahlungsschrägwinkel erfolgt, der von der Vertikalen zur örtlichen Oberfläche um wenige Grad abweicht. Der Bestrahlungsschrägwinkel kann in der Trennebene im Bereich von kleiner 90° bis 70°, bevorzugt im Bereich von 89,5° bis 75°, bevorzugter im Bereich 89° bis 80° und besonders bevorzugt im Bereich zwischen 85° und 80° zur Tangenten im Auftreffpunkt des Laserstrahls an das Glasrohr oder an das rohrförmige Glasvorprodukt liegen.
Es wurde festgestellt, dass wenn - entgegen der Erfindung - orthogonal zur Tangente der Rohroberfläche oder der Glasvorproduktoberfläche eingestrahlt wird, um durch Filamente die Bruchebene des Rohrglases oder des Glasvorproduktes zu bestimmen, beim Abtrennen von Abschnitten des Glasrohres oder Glasvorproduktes im Bereich der Innenwandung des Glasrohres oder Glasvorproduktes Absplitterungen oder Partikel größer 100 µm entstehen, was Waschprozesse und ggf. weitere Endbearbeitung des Glasrohres oder Glasvorproduktes wegen erhöhter Rauigkeit in der Bruchfläche bedingen würde. Überraschenderweise wurde festgestellt, dass bei Erzeugung der Filamente innerhalb der gewünschten Trennebene in einem Schrägwinkel zur örtlichen Oberfläche des Glasrohres oder Glasvorproduktes gemäß Erfindung die Bildung von Splitter und Partikel vermieden werden kann und dass eine glattere Bruchfläche erzielt wird als im Falle von orthogonalen Filamenten zur Oberfläche des Glasrohrs oder Glasvorproduktes. Der jeweils beste Wert des Bestrahlungsschrägwinkels wird in Abhängigkeit von Wanddicke und örtlichem Durchmesser des Glasrohrs oder Glasvorproduktes experimentell bestimmt. Es gilt: Je kleiner der Durchmesser, umso größer der Bestrahlungsschrägwinkel. So kann der Effekt der Vermeidung von Splitter und Partikel beim Abtrennen der Abschnitte des Glasrohres oder Glasvorproduktes durch geeignete Kombination von Bestrahlungsschrägwinkel und örtlichem Durchmesser maximiert werden.

In der Praxis wird der Bestrahlungsschrägwinkel dadurch erzielt, dass mit einem Versatz der fokussierten Strahlung gegenüber der radialen Richtung, gemessen auf der Oberfläche des Glasrohres oder Glasvorproduktes, gearbeitet wird. Mit anderen Worten ist die Einstrahlrichtung seitlich zur Achse des Glasrohres oder Glasvorproduktes versetzt. Für Glasrohre oder Glasvorprodukte im örtlichem Durchmesserbereich von 3 mm bis 50 mm, bevorzugt im Bereich 5 bis 40 mm, liegt der Versatz im Bereich von 0,1 mm bis 3 mm, bevorzugt im Bereich von 0,5 mm bis 2 mm. Es versteht sich, dass mit dem Versatz auch die Fokuslage der Fokussieroptik nachgestellt werden muss.

Bei der Herstellung von Glasprodukten aus rohrförmigen Glasrohrprodukten kann eine gewünschte Trennebene als Sollbruchstelle erzeugt werden, beispielsweise bei Ampullen als Glasprodukte.

Die Erfindung betrifft auch eine Vorrichtung zur Herstellung von rohrförmigen Glasvorprodukten oder von Glasprodukten. Eine solche Vorrichtung kann die Trennung von Rohrglas oder von Glasvorprodukt entlang einer gewünschten Trennebene in abgemessene Abschnitte betreffen. Die Vorrichtung umfasst eine Zufuhreinrichtung von Rohrglas oder von einem Glasvorprodukt, eine Laser-basierte Bestrahlungseinrichtung zur Erzeugung fokussierter Strahlung entlang einer gewünschten Trennebene in einem Bestrahlungsschrägwinkel zur örtlichen Oberfläche des Glasrohres oder Glasvorproduktes mittels einer Fokussieroptik, einer Führungseinrichtung, um die Fokussieroptik entlang einer gewünschten Trennebene in gewünschtem Abstand und in gewünschtem Bestrahlungsschrägwinkel zur Oberfläche des Glasrohres oder Glasvorprodukte stabil zu führen oder zu halten, und eine Abtransporteinrichtung. Mit der erfindungsgemäßen Vorrichtung erfolgt die Bestrahlung in der Trennebene zur Erzeugung der Filamente in einem von der Senkrechten abweichenden Bestrahlungsschrägwinkel zur örtlichen Oberfläche des Glasrohres oder Glasvorproduktes. Die Filamente verlaufen von außen nach innen. Das Abtrennen des abgemessenen Glasrohrabschnittes erfolgt in an sich bekannten Trennvorrichtungen, die das Glasrohr oder das Glasvorprodukt durch mechanisch oder thermisch eingebrachte Spannung spalten und so eine saubere Bruchfläche in der Trennebene des Glasrohres oder Glasvorproduktes schaffen, womit einwandfreie Abschnittsenden gewonnen werden.

Die Zufuhreinrichtung von Glasrohr oder von Glasvorprodukt ist dazu beschaffen, das Glasrohr oder das Glasvorprodukt schubweise oder kontinuierlich in die Richtung der Längsachse vorzuschieben, das heißt translatorisch zu bewegen. Gleichzeitig kann eine Rotation des Glasrohres oder Glasvorproduktes erfolgen, wenn die Laser-basierte Bestrahlungseinrichtung stationäre fokussierte Strahlung erzeugt. Bei stehendem Rohr wird um das Glasrohr oder das Glasvorprodukt rotierende fokussierte Strahlung erzeugt.

Die Zufuhreinrichtung kann auch einen Teil einer Glasrohr-Erzeugungseinrichtung oder einer Glasrohr-Formgebungseinrichtung darstellen, die das Glasrohr oder das Glasvorprodukt der Laser-basierten Bestrahlungseinrichtung, gegebenenfalls noch im heißen Zustand, zuführt. Geeignete Formungsprozesse für das Glasrohr oder das Glasvorprodukt sind Herstellungsverfahren nach Danner, Vello oder auch ein Overflow-Fusion-Prozess. Beim Prozess nach Danner wird ein Strom oder Strang geschmolzenen Glases auf eine schräg gelagerte, rotierende Spindel gegeben. Durch das Rotieren wird das Glas auf dem Umfang der Spindel verteilt, während es die Spindel entlang in Richtung auf deren unteres Ende fließt. Beim Vello-Prozess tritt das geschmolzene Glas durch eine ringförmige Düse aus. Der Durchmesser des so entstehenden Rohres kann durch Einbringen von Druckluft vergrößert und eingestellt werden.

Wenn am bewegten Glasrohr oder Glasvorprodukt gearbeitet werden soll, etwa wenn der Prozess an einem kontinuierlich, rohrförmig hergestellten Glaskörper direkt am Zug aus der Schmelze oder nach teilweisem Aufschmelzen eines Glaskörpers durchgeführt werden soll, dann wird bevorzugt, die Laser-basierte Bestrahlungseinrichtung mitlaufend zur Zufuhrrichtung des Glasrohres oder Glasvorproduktes auszubilden. Auf diese Weise ist es möglich, in der gewünschten Trennebene um das Glasrohr oder Glasvorprodukt herum die fokussierte Bestrahlung vorzunehmen, um das Glasrohr oder das Glasvorprodukt zur Trennung in Abschnitte vorzubereiten. Je nach Arbeitsprogramm erfolgt die Trennung unmittelbar nach der Vorbereitung der Trennung oder zu einem späteren Zeitpunkt. Es kann auch gemischt gearbeitet werden, etwa dass eine unmittelbare Trennung durchgeführt wird, der eine später zu erfolgende Trennung nachfolgt, so dass rohrförmige Körper abgetrennt werden, die einen Abschnitt mit vorbereiteter Sollbruchstelle aufweisen. Solche rohrförmigen Körper stellen Zwischenerzeugnisse bei der Herstellung von beispielsweise Ampullen dar, die mit einer Sollbruchstelle versehen sind. Gemäß einer Ausführungsform der Erfindung ist dementsprechend ein Abschnitt eines Glasrohres oder Glasvorproduktes vorgesehen, bei welchem zwischen den endseitigen Bruchflächen zumindest eine Sollbruchebene mit schräg zur örtlichen Radialen verlaufenden Filamenten vorgesehen ist. Die Sollbruchebene liegt vorzugsweise senkrecht zur Längsachse des rohrförmigen Abschnitts, es ist aber auch denkbar, dieses Ebene schräg zur Längsachse einzufügen, so dass die Längsachse die Ebene unter einem schrägen Winkel durchstößt.

Die Laser-basierte Bestrahlungseinrichtung kann einen Laser, eine Fokussieroptik und einen diese verbindenden Lichtleiter enthalten. Als Lichtleiter für die intensiven Laserpulse sind beispielsweise Hohlglasfasern geeignet. Die Führungseinrichtung kann Teil eines Roboters bilden, der die Fokussieroptik mit gewünschtem Abstand und gewünschter Winkelausrichtung zur Oberfläche des Glasrohres oder Glasvorproduktes in einer gewünschten Trennebene des Glasrohres oder Glasvorproduktes rund um das Glasrohr oder das Glasvorprodukt stabil zu führen vermag. Je nachdem, ob das Glasrohr oder das Glasvorprodukt während der Bearbeitung steht oder bewegt wird, kann der Roboterarm stationär oder mitlaufend zum Glasrohr oder Glasvorprodukt um dieses herumgeführt werden. In beiden Ausführungsformen kann die Fokussieroptik in einem Führungskopf enthalten sein, der einen Sensor zur Bestimmung des Abstandes zur Oberfläche des Glasrohres oder Glasvorproduktes und der Lage der erzeugten Filamente aufweist.

Bei einer weiteren Ausführungsform weist die Laser-basierte Bestrahlungseinrichtung einen drehbaren Scannerkopf mit Strahlführung zu der Fokussieroptik auf, die an der Innenseite eines Ringspiegels angeordnet ist, durch dessen Ringraum sich der zu bearbeitende Rohrkörper erstreckt. Wenn bewegtes Glasrohr oder Glasvorprodukt bearbeitet werden soll, muss bei dieser Ausführungsform die Laser-basierte Bestrahlungseinrichtung mitlaufend zum bewegten Glasrohr oder Glasvorprodukt sein. Wenn man dies vermeiden will und eine stationäre Bestrahlungseinrichtung vorsehen will, verwendet man einen Ringspiegel, der entlang einer Schraubspirale angeordnete Abschnitte aufweist. Die Bewegung des Glasrohres oder Glasvorproduktes wird mit dem Scannerumlauf der Bestrahlungseinrichtung abgestimmt, so dass die Filamente in der gewünschten Trennebene des Glasrohres oder Glasvorproduktes erzeugt werden.

Anstelle von Linsen können Abschnitte des Ringspiegels verwendet werden, die als Abbildungsoptik ausgebildet sind. Auch die Kombination von gekrümmten Spiegeln und Linsen als Abbildungsoptiken ist möglich.

Es können auch mehrere drehbare Scannerköpfe um das zu bearbeitende Glasrohr oder Glasvorprodukt angeordnet sein.

Die Erfindung betrifft auch nach dem erfindungsgemäßen Verfahren hergestellte Abschnitte aus Glasrohr oder Glasvorprodukt. Solche Abschnitte weisen am Abschnittsende eine Bruchfläche auf, die schräg zu den örtlichen Rohrradien verlaufende, aufgebrochene Filamente zeigt. Der erfindungsgemäße Glasrohrabschnitt oder Glasvorproduktabschnitte kann ein Zwischenprodukt darstellen, etwa auf dem Wege zu einem Endprodukt, beispielsweise eine Ampulle. Indem das Zwischenprodukt eine sauber ausgebildete Bruchfläche aufweist, die ohne Splitterungen und ohne Partikel größer 150 µm, bevorzugt größer 100µm, besonders bevorzugt größer 50 µm, ganz besonders bevorzugt größer 20 µm ist, ist die Weiterverarbeitung zu einem Endprodukt kostengünstiger und auch sicherer gegenüber Glasrohrabschnitten des Standes der Technik. Die Glasrohrabschnitte oder Glasvorproduktabschnitte haben vorzugsweise eine hohlzylindrische Form, deren ringförmige Stirnflächen durch die Bruchflächen gebildet werden. Der Querschnitt des Glasrohrabschnitts oder Glasvorproduktabschnittes kann aber auch elliptisch oder allgemein unrund sein.

Erfindungsgemäße Glasvorprodukte mit eingebauter Sollbruchstelle können auch Endprodukte darstellen, etwa als Ampulle, die eine vorbereitete Bruchfläche aufweist, welche vom Benutzer der Ampulle zu öffnen ist. Auch dabei ist es wünschenswert, wenn die Bruchfläche keine Absplitterungen oder Partikel größer 50 µm oder 100 µm aufweist.

Bei dem erfindungsgemäßen Glasrohrabschnitt oder Glasvorproduktabschnitt sollten die Filamente einen Abstand voneinander im Bereich von 2 bis 15 µm, bevorzugt 3 bis 12 µm, besonders bevorzugt im Bereich von 4 bis 8 µm, gemessen auf der Außen-, beziehungsweise Mantelfläche des Glasrohres oder Glasvorproduktes, einnehmen. Dabei können die Filamente zwei oder mehreren Filamentbereichen angehören, die um den Umfang des Glasrohres oder Glasvorproduktes verteilt angeordnet sind und durch unbetroffene Bereiche voneinander getrennt sind. Die Filamentbereiche enthalten jeweils eine Gruppe von Einzelfilamenten, die selbst voneinander beabstandet sind. Auf diese Weise wird der Aufwand bei der Herstellung der vorbereiteten Bruchstellen bedeutend reduziert.

Die unbetroffenen Bereiche zwischen den Filamentbereichen können eine in Umfangrichtung des Glasrohres oder Glasvorproduktes gemessene Ausdehnung von wenigstens 50 µm oder wenigstens 100 µm aufweisen. Die Anzahl der Filamentbereiche, um den Umfang des Glasrohres oder Glasvorproduktes verteilt, kann bis zu zwölf Bereiche betragen. Die Filamentbereiche sollten wenigstens 8%, bevorzugt wenigstens 16% und höchst bevorzugt wenigstens 32% des Umfangs des Glasrohres oder Glasvorproduktes einnehmen.

Die Erfindung betrifft auch die Verwendung von nach der Erfindung hergestellten Glasprodukten für Pharmaverpackungen, etwa von Ampullen.

### Kurzbeschreibung der Zeichnungen

Ausführungsbeispiele der Erfindung werden anhand der Zeichnung beschrieben. Dabei zeigt:
- Fig. 1: ein erstes Schema einer Bestrahlungseinrichtung zur Erzeugung von Filamenten in einem Glasrohr,
- Fig. 2: ein zweites Schema einer Bestrahlungseinrichtung mit einem drehbaren Scannerkopf,
- Fig. 3: ein drittes Schema einer Bestrahlungseinrichtung mit schraubförmigem Spiegel,
- Fig. 4: ein viertes Schema einer Bestrahlungseinrichtung mit Abbildungsoptik-Ringspiegel,
- Fig. 5: eine Bestrahlungseinrichtung mit Abtrennvorrichtung von Glasrohrabschnitten,
- Fig. 6: eine geometrische Darstellung der Ausrichtung der Bestrahlungseinrichtung zu dem zu bearbeitenden Glasrohr,
- Fig. 7: ein Schema eines Führungskopfes mit integrierter Bestrahlungseinrichtung,
- Fig. 8: eine Bestrahlungsoptik in dezentrierter Anordnung gegenüber der örtlichen Oberfläche eines Glasrohres,
- Fig. 9: ein vergrößerter Abschnitt einer Bruchfläche eines Glasrohres mit schrägverlaufenden Filamenten,
- Fig. 10: eine Ausschnittsvergrößerung zu Fig. 9,
- Fig. 11: eine nochmalige Ausschnittsvergrößerung zu Fig. 10, und
- Fig. 12: einen Glasrohrabschnitt mit Filamentbereichen.

### Detaillierte Beschreibung der Erfindung

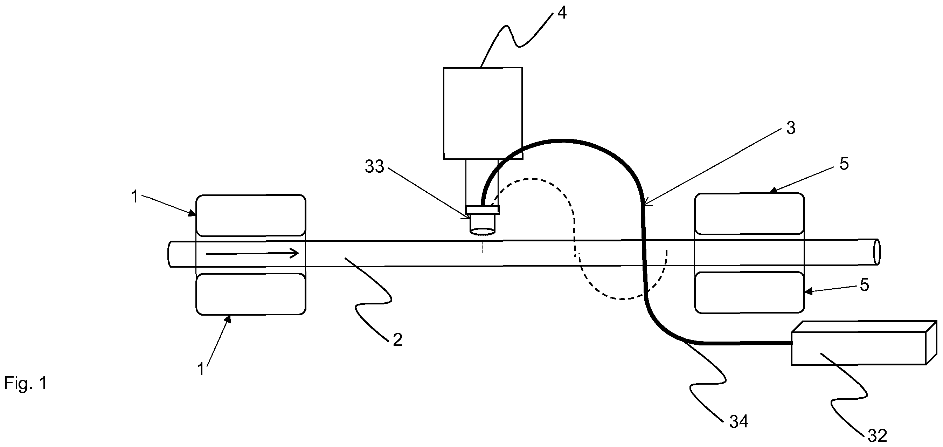

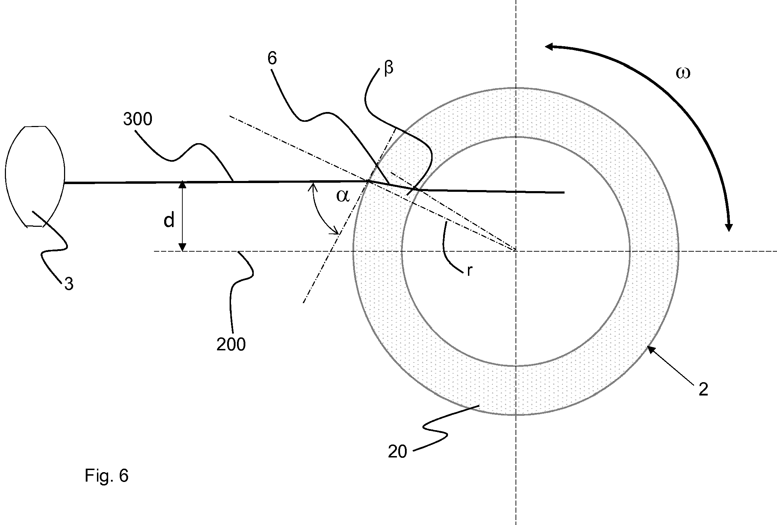

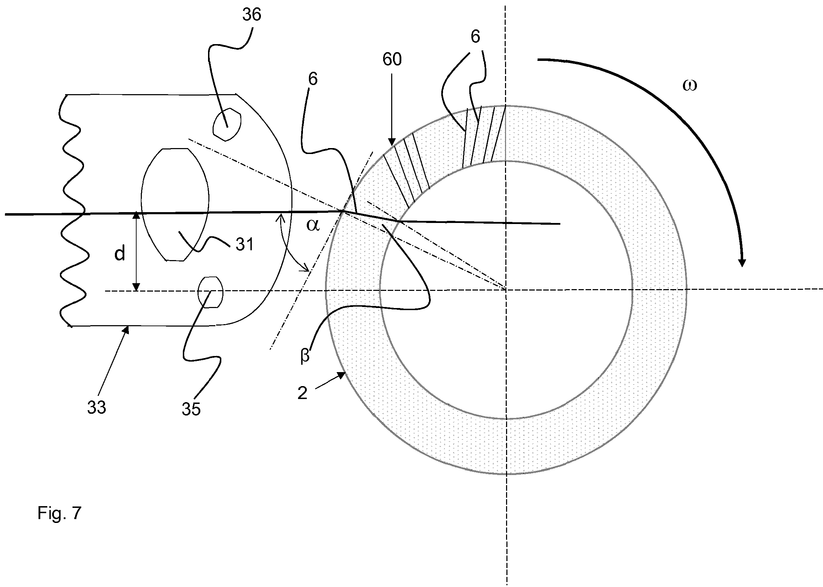

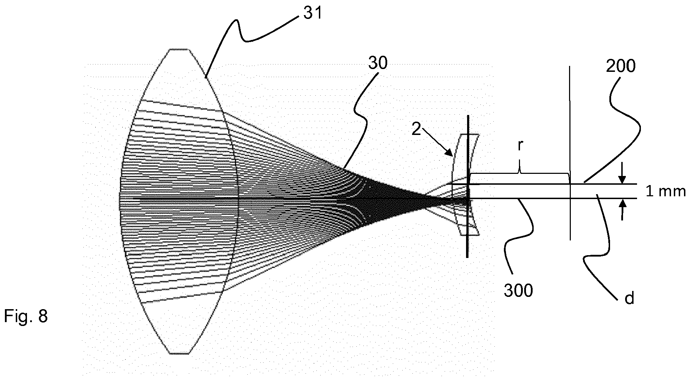

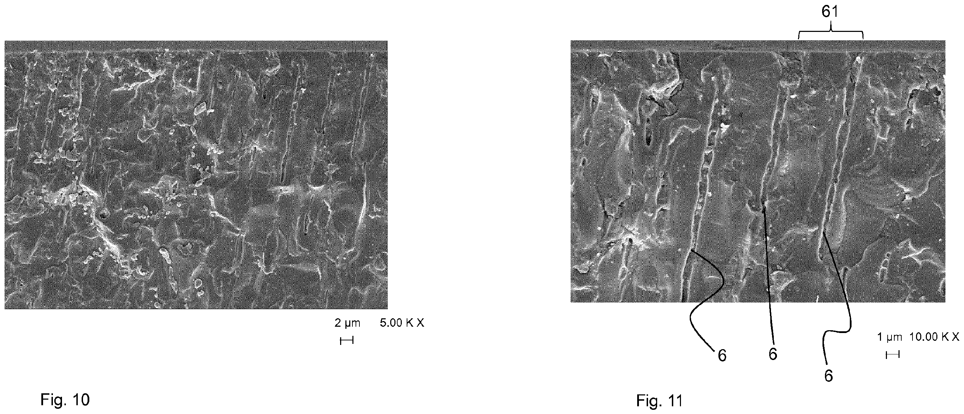

Fig. 1 zeigt eine Vorrichtung zur Vorbereitung der Trennung von Rohrglas in abgemessene Glasrohrabschnitte. Die Vorrichtung umfasst eine Zufuhreinrichtung 1 von Glasrohr 2, eine Laser-basierte Bestrahlungseinrichtung 3 zur Erzeugung fokussierter Strahlung 30 (Fig. 8) mittels einer Fokussieroptik 31 (Fig. 7), eine Führungseinrichtung 4, um die Fokussieroptik in gewünschten Abstand und in einem gewünschten Bestrahlungsschrägwinkel α (Fig. 6) zur Oberfläche des Glasrohres 2 stabil zu führen oder zu halten, sowie eine Abtransporteinrichtung 5 für Glasrohr. Die Laser-basierte Bestrahlungseinrichtung 3 umfasst einen Laser 32, einen Führungskopf 33 für die Fokussieroptik 31 und eine Hohlfaser 34, welche den Laser 32 mit der Fokussieroptik 31 verbindet. Der Führungskopf 33 enthält ferner Sensoren 35, 36 (Fig. 7) zur Bestimmung des Abstandes der Fokussieroptik 31 zur Oberfläche des Glasrohres 2 und zur Lage von erzeugten Filamenten 6 (Fig. 10 und 11). Der Führungskopf 33 wird von der Führungseinrichtung 4 geführt, der Teil eines Roboters bilden kann, so dass die Fokussieroptik 31 innerhalb des Führungskopfes 33 mit gewünschtem Abstand und gewünschter Winkelausrichtung zur Oberfläche des Glasrohres 2 und in einer gewünschten Trennebene des Glasrohres rund um das Glasrohr geführt werden kann.

Ein geeigneter Laser für eine erfindungsgemäße Vorrichtung ist ein Neodym-dotierter Yttrium-Aluminium-Granat-Laser mit einer Wellenlänge von 1064 Nanometern, der auch frequenzverdoppelt arbeiten kann. Dabei ist die geeignete Pulsdauer eines Laserpulses vorzugsweise kürzer als 100 Pikosekunden, bevorzugt kürzer als 10 Pikosekunden.

Fig. 6 zeigt das Schema der Erzeugung von Filamenten 6 in der Trennebene 20 des Glasrohres 2. Eingezeichnet sind der zur Oberfläche des Glasrohres 2 senkrechte Radiusvektor 200 und die Bestrahlungsachse 300 der fokussierten Strahlung 30, die einen parallelen Versatz d zueinander haben. Dadurch ergibt sich der Bestrahlungsschrägwinkel a. Wegen der Brechung in Glas wird die Bestrahlungsachse zur Rohrachse hin gebrochen, woraus sich ein Winkel β zwischen örtlichem Radiusvektor r und örtlicher Bestrahlungsachse ergibt. Ferner sollen sich das Glasrohr 2 und die Bestrahlungseinrichtung 3 relativ zueinander in Schritten mit einer Winkelgeschwindigkeit ω drehen.

Fig. 7 zeigt eine analoge Ansicht zu Fig. 6, jedoch mit Gruppen 60 von Filamenten 6, die nach entsprechenden Schrittdrehungen des Glasrohres 2 (bei geänderter Anordnung des Führungskopfes 33) erzeugt worden sind. Ferner ist eine schematische Darstellung des Führungskopfes 33 mit der Fokussieroptik 31 und den Sensoren 35, 36 skizziert, von denen der Sensor 35 als Abstandssensor zur Bestimmung des Abstandes der Fokussieroptik 31 von dem Glasrohr 2 und der Sensor 36 als Abtastsensor zu Feststellung von erzeugten Filamenten 6 dienen.

Fig. 8 zeigt eine vergrößerte Computerdarstellung der fokussierten Strahlung 30 zur Erzeugung von Filamenten 6 in einem Glasrohr 2 mit Innenradius r. Der Versatz d zwischen Radiusvektor 200 und Bestrahlungsachse 300 beträgt für ein Glasrohr des Innenradius r = 6 mm in etwa 1 mm.

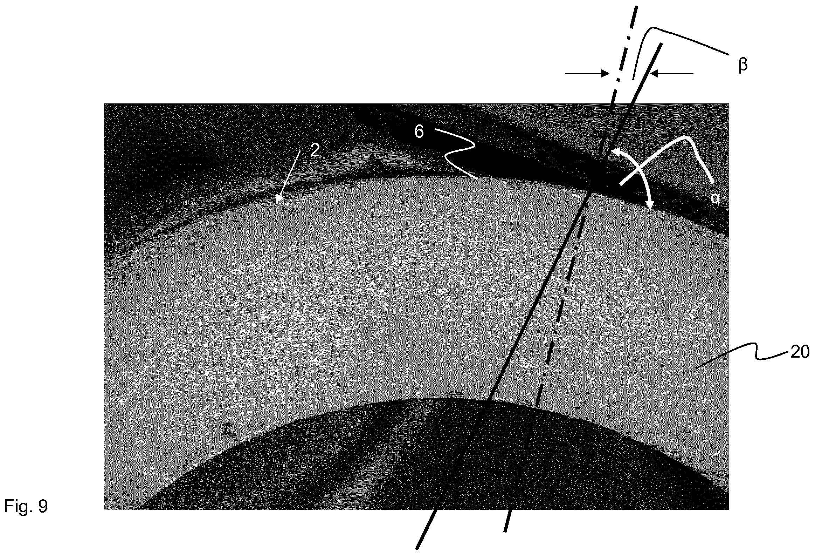

Fig. 9 zeigt eine vergrößerte fotografische Aufnahme der Trennebene 20 als Bruchfläche mit einer Vielzahl von Filamenten 6, die im Winkel β schräg zu dem örtlichen Radialvektor verlaufen.

Die Fig. 10 und 11 zeigen nochmalige fotografische Vergrößerungen der Trennebene 20 als einer Bruchfläche mit geöffneten Filamenten 6, die im Abstand 61 voneinander verlaufen.

Solche Filamente 6 sind mit einer laserbasierten Bestrahlungseinrichtung 3 erzeugt worden, die eine Bikonvexlinse mit 16 mm Brennweite und einer Apertur von 18 mm umfasste, der Laser gab Strahlung mit einer Wellenlänge von 1064 nm, einer Pulsenergie > 200 µJ, einer Burstenergie von 100 µJ bei 4 Bursts und einer Burstfrequenz von 50 MHz ab. Der Pitch betrug 8µm, die Fokuslage -1,25 mm und der Versatz d = 1 mm. Das Glasrohr hatte 6,85 mm Außendurchmesser und eine Wanddicke von ca. 1 mm.

Die Zufuhreinrichtung 1 kann eine Glas-Zugeinrichtung aus der Schmelze oder eine Wiederzieheinrichtung darstellen. Es kann aber auch eine diskontinuierlich betriebene Zufuhreinrichtung benutzt werden. Je nachdem, ob das Glasrohr 2 beim Anbringen der Filamente 6 stationär oder in Längsbewegung ist, vollführt der Führungskopf 33 im Raum eine Rotationsbewegung oder eine Schraubbewegung, jedoch immer in Ausrichtung auf die Oberfläche des Glasrohres 2. Die Rotationsbewegung oder die Schraubbewegung des Führungskopfes 33 ist in Bezug zu der gewünschten Trennebene 20 als ein orbitaler Umlauf der Bestrahlungseinrichtung 3 anzusehen. Die Orbitalbewegung ist auf die Bewegung des Glasrohres 2 so abgestimmt, dass die von der Bestrahlungseinrichtung abgegebene und durch den Führungskopf 33 geführte Strahlung 30 immer in der gewünschten Trennebene 20 verläuft. Die fokussierte Strahlung 30 trifft dabei nicht zentrisch auf das Glasrohr 2, sondern mit einem gewissen Versatz d zur Radialen (Fig. 6), so dass sich ein Bestrahlungsschrägwinkel α zur örtlichen Tangente an das Glasrohr ergibt, der im Bereich von kleiner 90° bis 70°, bevorzugt im Bereich von 89,5° bis 75°, bevorzugter 89° bis 80°, besonders bevorzugt zwischen 85° und 80° liegen kann. Der optimal Bestrahlungsschrägwinkel α hängt vom Rohrdurchmesser und den optischen Eigenschaften des Materials ab. Der jeweils günstigste Wert wird durch Tests oder Berechnungen ermittelt.

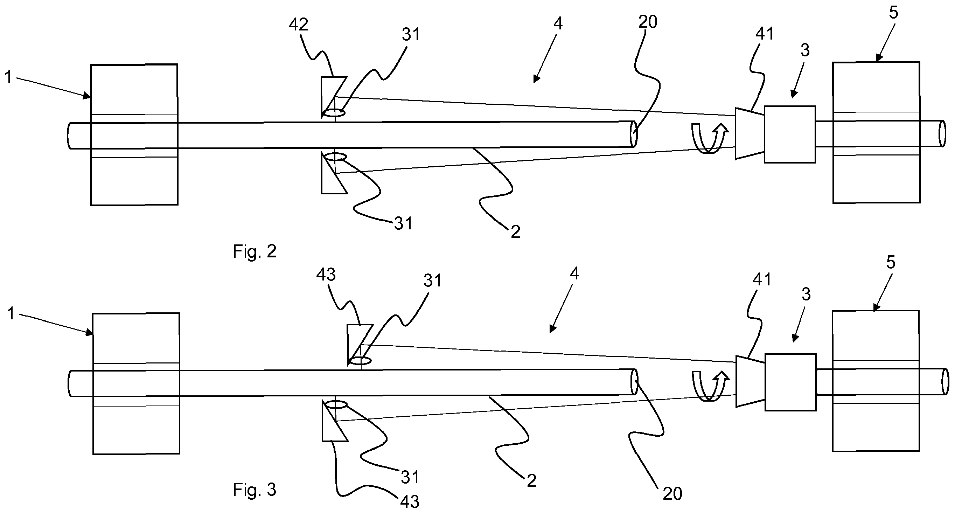

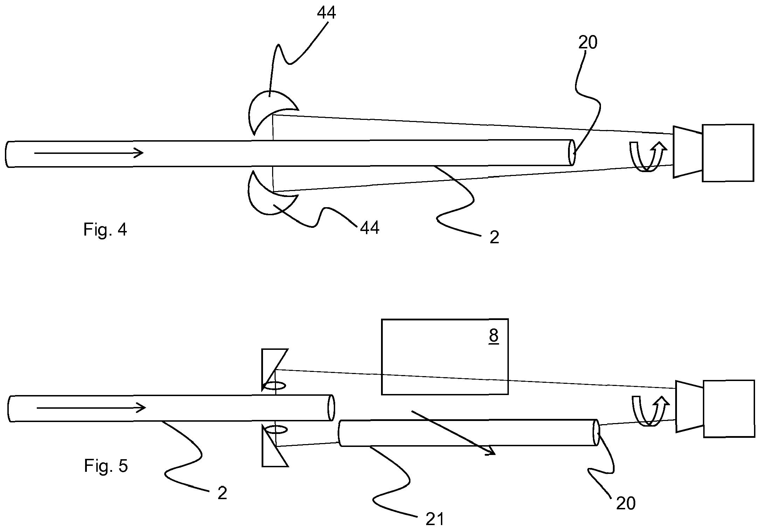

Fig. 2 zeigt eine Ausführungsform der Vorrichtung zur Vorbereitung der Trennung von Rohrglas in abgetrennte Glasrohrabschnitte 21 (Fig. 5), bei der die Laser-basierte Bestrahlungseinrichtung 3 einen drehbaren Scannerkopf 41 mit Strahlführung zu der Fokussieroptik 31 aufweist, die an der Innenseite eines Ringspiegels 42 angeordnet ist, durch dessen Ringraum sich das zu bearbeitende Glasrohr 2 erstreckt. Der drehbare Scannerkopf 41 und der Ringspiegel 42 bilden zusammen eine Führungseinrichtung 4 für die Fokussieroptik 31. Die Bestrahlungseinrichtung 3 enthält ein oder mehrere Laser (nicht gezeigt), um eine oder mehrere Laserstrahlen gleichzeitig der Fokussieroptik 31 zuzuführen. Der Ringspiegel 42 weist eine mit Facetten versehene Innenseite auf, die zu dem Glasrohr 2 hin geneigt ist, das sich durch den Ringraum des Ringspiegels 42 erstreckt. Die Facetten sorgen dafür, dass ein Bestrahlungsschrägwinkel α eingehalten wird.

Die Ausführungsform der Vorrichtung nach Fig. 2 ist für den stationären Betrieb konstruiert. Das Glasrohr 2 wird abschnittsweise vorgeschoben und arretiert, wonach durch Betrieb der Bestrahlungseinrichtung 3 die Filamente 6 in einer gewünschten Trennebene 20 des Glasrohres 2 erzeugt werden. Die Filamente 6 verlaufen schräg, wie in den Fig. 9 bis 11 dargestellt.

Die Ausführungsform der Fig. 2 lässt sich auch für den fortlaufenden Betrieb bauen. Der Abstand zwischen der Zufuhreinrichtung 1 und der Glasrohrabtransporteinrichtung 5 wird auf wenigstens das Doppelte des in Fig. 2 dargestellten Abstandes vergrößert. Ferner werden die Laser-basierte Bestrahlungseinrichtungen 3 und die Führungseinrichtung 4 mitlaufend zur Zufuhreinrichtung 1 von Glasrohr 2 ausgebildet. Auf diese Weise können während des Mitlaufs die Filamente 6 in der gewünschten Trennebene erzeugt werden. Danach wird die Führungseinrichtung in ihre Ausgangslage zurückverschoben, um sogleich mit dem vorrückenden Glasrohr weiter nach vorn zu wandern.

Die Ausführungsform nach Fig. 3 der Vorrichtung stellt eine Abwandlung zur Ausführungsform der Fig. 2 dar. Der Spiegel weist entlang einer Schraube angeordnete Spiegelabschnitte 43 auf. Das Glasrohr 2 kann kontinuierlich vorgeschoben und dabei mit den Filamenten 6 in der gewünschten Trennebene versehen werden. Die Spiegelabschnitte 43 werden nacheinander mit dem Laserstrahl beaufschlagt, angepasst an die Vorschubgeschwindigkeit des Glasrohres derart, dass die Filamente 6 in der gewünschten Trennebene erzeugt werden.

Fig. 4 zeigt eine Ausführungsform der Vorrichtung, bei der der Ringspiegel Abschnitte 44 aufweist, die als Abbildungsoptik ausgebildet sind. Die Abbildungsoptik eignet sich für Rohrglas mit Radien größer als 10 mm. Wenn die Radien kleiner als 10 mm sind, wird noch eine Zwischenoptik (nicht gezeigt) in der Art der Fokussieroptik 31 der Fig. 2 und 3 eingesetzt, womit der Einkopplungswinkel der Laserbestrahlung größer gemacht werden kann und sich das Filament 6 im Inneren des Glasrohres 2 deutlicher ausprägt als ohne diese Maßnahme.

Eine Zwischenoptik zwischen der Fokussieroptik 31 und der Glasrohroberfläche kann auch bei den Ausführungsformen der Fig. 1 bis 3 angewandt werden. Als Zwischenoptik kann auch eine Immersionsflüssigkeit mit hohem Brechungsindex relativ zur Luft verwendet werden, wo dies angängig ist.

Fig. 5 zeigt eine weitere Ausführungsform unter Fortlassung der Zufuhreinrichtung und der Abtransporteinrichtung des Glasrohres 2. Der Aufbau entspricht der Ausführungsform nach Fig. 2. Das Glasrohr 2 wird abschnittsweise vorgeschoben und die Trennebene mit den Filamenten 6 markiert. Das Abtrennen des abgemessenen Glasrohrabschnittes wird jedoch noch in der Vorrichtung vorgenommen. Hierzu dient eine Abtrenneinrichtung 8, die einen mechanischen Druck auf das Glasrohr 2 oder einen Kälteschock an der Trennstelle ausübt, so dass ein Glasrohrabschnitt 21 von dem zugeführten Glasrohr 2 abgetrennt wird. Der abgetrennte Glasrohrabschnitt 21 wird durch nicht gezeigte Mittel abtransportiert.

Die entlang der Trennebene 20 erzeugte Bruchfläche zeigt eine gewisse Rauigkeit, wie den fotografischen Darstellungen der Fig. 9 bis 11 zu entnehmen ist. Die Bruchfläche ist aber partikelfrei, wenn Absplitterungen größer 150 µm oder 100 µm, oder 50 µm oder sogar nur 20 µm unter "Partikel" verstanden werden. Als markante Spuren in der Bruchfläche sind schräg verlaufende, aufgebrochene Filamente 6 zu erkennen. Die Filamente 6 stellen vom Laser erzeugte kanalförmige Perforationen oder Teilperforationen in der Rohrwandung des Glasrohres 2 dar. Der Schrägungswinkel β (Fig. 6) des Verlaufs der Filamente 6 zu dem örtlichen Rohrradius r liegt im Bereich von 1° bis 15°, wobei für Rohre mit größerem Durchmesser die kleineren Winkel und für Rohre mit kleinerem Durchmesser die größeren Winkel anzutreffen sind. Am vorteilhaftesten haben sich Winkelwerte im Bereich von 5° bis 10° erwiesen. Die Filamente 6 haben einen Filamentabstand 61 voneinander, der im Bereich von 2 µm bis 15 µm liegt. Ein Abstandsbereich von 4 µm bis 8 µm wird bevorzugt.

Die Bruchfläche braucht nicht durchgängig mit Filamenten 6 belegt sein, wie in Fig. 7 dargestellt. Filamentbereiche 60 können sich mit unbetroffenen Bereichen abwechseln. Die Filamentbereiche 60 enthalten jeweils eine Gruppe von einzelnen Filamenten 6, die selbst voneinander beabstandet sind, wie zuvor erwähnt. Die unbetroffenen Bereiche zwischen den Filamentbereichen 60 können eine im Umfangsrichtung des Rohres gemessene Ausdehnung von wenigstens 50 µm oder von wenigstens 100 µm einnehmen. Die Anzahl der Filamentbereiche 60 (und demnach auch der unbetroffenen Bereiche) beträgt bis zu zwölf Bereiche, die um den Umfang des Glasrohrs verteilt angeordnet sind. Die Filamentbereiche 60 nehmen wenigstens 8 %, bevorzugt wenigstens 16 % bzw. zumeist bevorzugt 32 % oder mehr des Umfangs des Glasrohres ein. Damit kann die Trennung von Rohrglas in Glasrohrabschnitte mit wenig Energieaufwand durchgeführt werden und trotzdem saubere Bruchflächen erzielt werden.

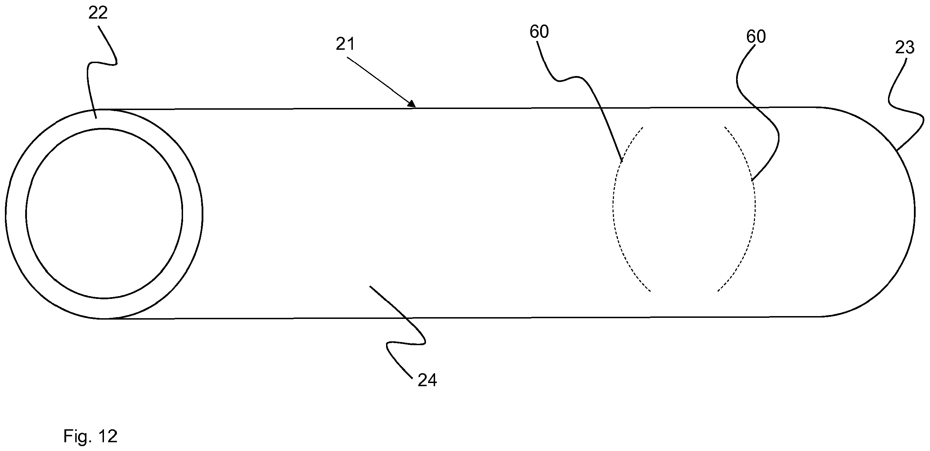

Fig. 12 zeigt einen Glasrohrabschnitt 21 als Beispiel eines Glasvorproduktes zur Erzeugung einer Ampulle. Der Glasrohrabschnitt zeigt durch Trennen erzeugte Abschnittsenden 22, 23 und Filamentbereiche 60 im vorbestimmten Abständen von diesen Abschnittsenden 22, 23. Die Filamentbereiche 60 nehmen beispielsweise zwei 120°-Segmente ein, die sich gegenüberliegen und deren Enden je 60°-Abstand voneinander haben. Zur Erzeugung einer Ampulle wird ein Glasmantelbereich 24 zwischen dem Abschnittsende 22 und den Filamentbereichen 60 warm ausgedehnt, und die Abschnittsenden 22, 23 werden zur Schließung vorbereitet, wonach die Ampulle gefüllt und verschlossen wird.

Es ist dem Fachmann ersichtlich, dass die vorstehend beschriebenen Ausführungsformen beispielhaft zu verstehen sind und die Erfindung nicht auf diese beschränkt ist, sondern in vielfältiger Weise variiert werden kann, ohne den Schutzbereich der Ansprüche zu verlassen. Ferner ist ersichtlich, dass die Merkmale unabhängig davon, ob sie in der Beschreibung, den Ansprüchen, den Figuren oder anderweitig offenbart sind, auch einzeln wesentliche Bestandteile der Erfindung definieren, selbst wenn sie zusammen mit anderen Merkmalen gemeinsam beschrieben sind.

### Bezugszeichenliste

- 1: Zufuhreinrichtung
- 2: Glasrohr
- 20: Trennebene
- 21: Glasrohrabschnitt
- 22, 23: Abschnittsende
- 24: Glasmantelbereich
- 200: Radiusvektor
- 3: Bestrahlungseinrichtung
- 30: fokussierte Strahlung
- 31: Fokussieroptik
- 32: Laser
- 33: Führungskopf
- 34: Hohlfaser
- 35: Abstandssensor
- 36: Abtastsensor
- 300: Bestrahlungsachse
- 4: Führungseinrichtung
- 41: Scannerkopf
- 42: Ringspiegel
- 43: Spiegelabschnitt
- 44: Ringspiegel-Abschnitt
- 5: Abtransporteinrichtung
- 6: Filament
- 60: Filamentbereich
- 61: Filamentabstand
- 8: Abtrenneinrichtung

## Patentansprüche

1. Verfahren zur Herstellung von Glasvorprodukten und von Glasprodukten, mit folgenden Schritten:
- Bereitstellen von Glasrohr (2) oder von rohrförmigem Glasvorprodukt,
- Laser-basiertes Bestrahlen des Glasrohres (2) oder des Glasvorproduktes mit fokussierter Strahlung (30) zur Erzeugung von Filamenten (6) in einer gewünschten Trennebene (20) des Glasrohres (2) oder des Glasvorproduktes;
- wobei die Bestrahlung mit fokussierter Strahlung (30) in der Trennebene (20) zur Erzeugung der Filamente (6) in einem von der Senkrechten abweichenden Bestrahlungsschrägwinkel (a) zur örtlichen Oberfläche des Glasrohres (2) oder des Glasvorproduktes erfolgt, wobei die Filamente von außen nach innen verlaufen,

2. Verfahren nach Anspruch 1,
wobei abgemessene Abschnitte (21) des Glasrohres (2) oder des Glasvorprodukts durch mechanisch oder thermisch eingebrachte (Zug-)Spannung in der Trennebene (20) des Glasrohres (2) oder des Glasvorprodukts abgetrennt und eine Bruchfläche entlang der Trennebene (20) erzeugt wird.

3. Verfahren nach Anspruch 1 oder 2,
wobei das Glasrohr (2) oder das Glasvorprodukt und die Bestrahlungseinrichtung mit fokussierter Strahlung (30) sich relativ zueinander so drehen, dass die fokussierte Strahlung (30) auf das Glasrohr (2) oder das Glasvorprodukt in der gewünschten Trennebene (20) auftrifft.

4. Verfahren nach einem der Ansprüche 1 bis 3, mit zumindest einem der folgenden Merkmale:
- der Bestrahlungsschrägwinkel (a) liegt im Bereich von 89,5° bis 75°, bevorzugt im Bereich zwischen 85° und 80°
- für Glasrohr (2) oder für Glasrohrvorprodukt im Durchmesserbereich von 3 mm bis 50 mm infolge des Bestrahlungsschrägwinkels α erfolgt ein Versatz (d) der fokussierten Strahlung (30) gegenüber der örtlich radialen Richtung, gemessen auf der Oberfläche des Glasrohres oder des Glasvorproduktes, im Bereich von 0,1 mm bis 3 mm.

5. Vorrichtung zur Herstellung von rohrförmigen Glasvorprodukten oder von Glasprodukten, umfassend:
- eine Zufuhreinrichtung (1) von Glasrohr (2) oder von Glasvorprodukt,
- eine Laser-basierte Bestrahlungseinrichtung (3) zur Erzeugung von fokussierter Strahlung (30) entlang einer gewünschten Trennebene (20) und in einem Bestrahlungsschrägwinkel (a) zur örtlichen Oberfläche des Glasrohres (2) oder des Glasvorproduktes mittels einer Fokussieroptik (31),
- eine Führungseinrichtung (4), um die Fokussieroptik (31) entlang einer gewünschten Trennebene (20) im gewünschten Abstand und im gewünschten Bestrahlungsschrägwinkel (a) zur Oberfläche des Glasrohres oder des Glasvorproduktes zu führen, um Filamente zu erzeugen, wobei die Filamente von außen nach innen verlaufen, sowie
- eine Abtransporteinrichtung (5) für abgemessene Glasrohrabschnitte (21) oder Glasvorprodukte.

6. Vorrichtung nach Anspruch 5, **gekennzeichnet durch** zumindest eines der folgenden Merkmale:
- die Führungseinrichtung (4) ist zum orbitalen Umlauf der Laser-basierten Bestrahlungseinrichtung (3) um das Glasrohr (2) oder das Glasvorprodukt ausgebildet
- die Laser-basierte Bestrahlungseinrichtung (3) und die Führungseinrichtung (4) sind mitlaufend zur Zufuhrrichtung von Glasrohr (2) oder Glasvorprodukt ausgebildet,
- die Zufuhreinrichtung (1) bildet Teil einer Erzeugungseinrichtung oder einer Formgebungseinrichtung für Glasrohr oder Glasvorprodukt.
sind.

7. Vorrichtung nach einem der Ansprüche 5 oder 6, mit zumindest einem der folgenden Merkmale:
- die Laser-basierte Bestrahlungseinrichtung (3) einen Laser (32), eine Fokussieroptik (31) und eine diese verbindende Hohlglasfaser (34) enthält und wobei die Führungseinrichtung (4) Teil eines Roboters bildet, der die Fokussieroptik (31) mit gewünschtem Abstand und gewünschter Winkelausrichtung zur Oberfläche des Glasrohres (2) oder des Glasvorproduktes in einer gewünschten Trennebene rund um das Glasrohr (2) oder Glasvorprodukt wirksam werden lässt,
- die Fokussieroptik (31) ist in einem Führungskopf (33) enthalten, der einen AbstandsSensor (35) zur Bestimmung des Abstandes zur Oberfläche des Glasrohres (2) oder des Glasvorproduktes und einen Abtast-Sensor (36) zur Bestimmung der Lage von erzeugten Filamenten (6) aufweist
- die Laser-basierte Bestrahlungseinrichtung (3) weist einen drehbaren Scannerkopf (41) mit Strahlführung zu der Fokussieroptik (31) auf, die an der Innenseite eines Spiegels (42) angeordnet ist, der einen Ringraum bildet, durch den sich das mit Filamenten (6) zu versehene Rohrglas (2) oder Glasvorprodukt erstreckt.

8. Vorrichtung nach dem vorstehenden Anspruch, mit zumindest einem der weiteren Merkmale:
- der Spiegel weist entlang einer Schraube angeordnete Abschnitte (43) auf,
- Abschnitte des Spiegels sind als Abbildungsoptik (44) ausgebildet,
- mehrere drehbare Scannerköpfe sind um das zur bearbeitende Glasrohr angeordnet.

9. Rohrförmiges Glasvorprodukt oder Glasprodukt, herstellbar nach einem der Ansprüche 1 bis 4, in Form von Rohrglas (2),
wobei in der gewünschten Trennebene (20) Filamente (6) angebracht sind, die schräg zu den jeweilig örtlichen Wandradien und von außen nach innen verlaufen.

10. Rohrförmiges Glasvorprodukt oder Glasprodukt, herstellbar nach einem der Ansprüche 1 bis 4, in Form von Ampullen, Karpulen oder Spritzenkörpern, deren Körper aus Rohrglas umgeformt sind,
wobei in der gewünschten Trennebene (20) Filamente (6) angebracht sind, die schräg zu den jeweilig örtlichen Wandradien und von außen nach innen verlaufen.

11. Rohrförmiges Glasvorprodukt oder Glasprodukt nach Anspruch 10, deren Körper an der gewünschten Trennebene (20) jeweils mit einer Sollbruchstelle zum späteren Abtrennen versehen sind.

12. Glasrohrabschnitt oder Glasvorproduktabschnitt, hergestellt mit dem Verfahren nach einem der Ansprüche 2 bis 4,
wobei eine erzeugte Bruchfläche am Abschnittsende schräg zu den jeweilig örtlichen Wandradien verlaufende, aufgebrochene Filamente (6) zeigt.

13. Glasrohrabschnitt oder Glasvorproduktabschnitt oder Glasproduktabschnitt nach Anspruch 12, **gekennzeichnet durch** zumindest eines der folgenden Merkmale:
- die Filamente (6) nehmen einen Abstand (61) voneinander im Bereich von 2 µm bis 15 µm, bevorzugt im Bereich von 3 µm bis 12 µm und besonders bevorzugt im Bereich von 4 µm bis 8 µm ein,
- die Filamente (6) gehören zwei oder mehreren Filamentbereichen (60) an, die um den Umfang des Glasrohres oder des Glasvorproduktabschnittes, oder des Glasproduktabschnittes verteilt angeordnet sind und durch unbetroffene Bereiche voneinander getrennt sind, und wobei die Filamentbereiche (60) jeweils eine Gruppe von Einzelfilamenten enthalten, die selbst voneinander beabstandet sind, wobei die unbetroffenen Bereiche zwischen den Filamentbereichen (60) vorzugsweise eine in Umfangrichtung des Produkts gemessene Ausdehnung von wenigstens 50 µm oder vorzugsweise wenigstens 100 µm aufweisen.

14. Glasrohrabschnitt oder Glasvorproduktabschnitt oder Glasproduktabschnitt nach vorstehendem Anspruch, mit zumindest einem der folgenden Merkmale:
- um den Umfang des Glasrohres oder des Glasvorproduktabschnittes, oder Glasproduktabschnittes sind bis zu zwölf Filamentbereiche (60) verteilt,
- die Filamentbereiche (60) nehmen wenigstens 8 %, bevorzugt wenigstens 16 % oder 32 % des Umfanges des Glasrohres, oder des Glasvorproduktabschnittes, oder des Glasproduktabschnittes ein,
- zwischen endseitigen Bruchflächen ist zumindest eine Sollbruchebene mit schräg zur örtlichen Radialen verlaufenden Filamenten vorhanden.

15. Verwendung von Glasrohrabschnitten oder Glasvorprodukten oder Glasprodukten nach einem der Ansprüche 12 bis 14 für Pharmaverpackungen.

## Claims

1. A method for producing glass preliminary products and glass products, comprising the steps of:
- providing glass tubing (2) or tubular glass preliminary product;
- laser-based irradiation of the glass tubing (2) or glass preliminary product with focused radiation (30) to produce filaments (6) in a desired separation plane (20) of the glass tubing (2) or of the glass preliminary product;
- wherein said irradiation with focused radiation (30) in the separation plane (20) for producing the filaments (6) is executed at an oblique irradiation angle (α) deviating from the perpendicular to the local surface of the glass tubing (2) or glass preliminary product; wherein said filaments extend inward from outside.

2. The method according to claim 1,
wherein sized portions (21) of the glass tubing (2) or of the glass preliminary product are separated by mechanically or thermally induced (tensile) stress in the separation plane (20) of the glass tubing (2) or of the glass preliminary product, and a fracture surface is produced along the separation plane (20).

3. The method according to claim 1 or 2,
herein the glass tubing (2) or the glass preliminary product and the irradiation device with focused radiation (30) rotate relative to each other such that the focused radiation (30) is incident on the glass tubing (2) or glass preliminary product in the desired separation plane (20).

4. The method according to any one of claims 1 to 3, comprising at least one of the following features:
- the oblique irradiation angle (α) is in a range from 89.5° to 75°, preferably in a range between 85° and 80°;
- for glass tubing (2) or glass tube preliminary product having a diameter in a range from 3 mm to 50 mm, an offset (d) of the focused radiation (30) relative to the local radial direction due to the oblique irradiation angle α is in a range from 0.1 mm to 3 mm measured on the surface of the glass tubing or of the glass preliminary product,

5. An apparatus for producing tubular glass preliminary products or glass products, comprising:
- a feeding device (1) for glass tubing (2) or glass preliminary product;
- a laser-based irradiation device (3) for generating focused radiation (30) along a desired separation plane (20) and at an oblique irradiation angle (α) relative to the local surface of the glass tubing (2) or of the glass preliminary product using focusing optics (31);
- a guiding device (4) for guiding the focusing optics (31) along a desired separation plane (20) at a desired distance and in the desired oblique irradiation angle (α) with respect to the surface of the glass tubing or of the glass preliminary product for producing filaments, which filaments extend inward from outside; and
- a take-off device (5) for sized glass tube portions (21) or glass preliminary products.

6. The apparatus according to claim 5, **characterized by** at least one of the following features:
- the guiding device (4) is configured for orbital revolution of the laser-based irradiation device (3) around the glass tubing (2) or glass preliminary product;
- the laser-based irradiation device (3) and the guiding device (4) are configured for moving concomitantly along the feeding direction (1) of glass tubing (2) or glass preliminary product;
- the feeding device (1) forms part of a fabrication apparatus or shaping apparatus for glass tubing or glass preliminary product.

7. The apparatus according to any one of claims 5 or 6, comprising at least one of the following features:
- the laser-based irradiation device (3) comprises a laser (32), focusing optics (31) and a hollow glass fiber (34) connecting them, and wherein the guiding device (4) forms part of a robot which causes the focusing optics (31) to be effective at the desired distance and the desired angular orientation relative to the surface of the glass tubing (2) or glass preliminary product in a desired separation plane around the glass tubing (2) or glass preliminary product;
- the focusing optics (31) is accommodated in a guide head (33) that includes a distance sensor (35) for determining the distance to the surface of the glass tubing (2) or of the glass preliminary product, and a scanning sensor (36) for determining the position of produced filaments (6);
- the laser-based irradiation device (3) comprises a rotatable scanner head (41) with beam guidance to the focusing optics (31) which is arranged on the inner surface of a mirror (42) that defines an annular space through which the glass tubing (2) or glass preliminary product to be provided with filaments (6) extends.

8. The apparatus according to the preceding claim, comprising at least one of the following further features:
- the mirror has portions (43) arranged along a helix;
- portions of the mirror are configured as imaging optics (44);
- a plurality of rotatable scanner heads are arranged around the glass tubing to be processed.

9. A tubular glass preliminary product or glass product, producible according to any one of claims 1 to 4, in the form of glass tubing (2), which includes filaments (6) that are provided in the desired separation plane (20) and extending inward from outside, obliquely to the respective local wall radii.

10. A tubular glass preliminary product or glass product, producible according to any one of claims 1 to 4, in the form of ampoules, cartridges, or syringe bodies, each having a body formed from glass tubing, which includes filaments (6) that are provided in the desired separation plane (20) and extend inward from outside, obliquely to the respective local wall radii.

11. The tubular glass preliminary product or glass product according to claim 10, the body of which is provided with a respective predetermined breaking point along the desired separation plane (20), for later separation.

12. A glass tube portion or glass preliminary product portion, produced by the method according to any one of claims 2 to 4, wherein a fracture surface produced at the end of the portion exhibits broken open filaments (6) that extend obliquely to the respective local wall radii.

13. The glass tube portion or glass preliminary product portion or glass product portion according to claim 12, **characterized by** at least one of the following features:
- the filaments (6) are arranged with a spacing (61) in a range from 2 µm to 15 µm, preferably in a range from 3 µm to 12 µm, and most preferably in a range from 4 µm to 8 µm to each other;
- the filaments (6) belong to two or more filament areas (60) that are distributed around the perimeter of the glass tube or of the glass preliminary product portion, or of the glass product portion and separated from each other by unaffected areas, and wherein each filament area (60) comprises a set of individual filaments which in turn are spaced apart from each other; wherein the unaffected areas between the filament areas (60) have an extent of at least 50 µm or at least 100 µm, measured in the circumferential direction of the product.

14. The glass tube portion or glass preliminary product portion or glass product portion according to the preceding claim, comprising at least one of the following features:
- up to twelve filament areas (60) are distributed around the perimeter of the glass tube or glass preliminary product portion or glass product portion;
- the filament areas (60) occupy at least 8 %, preferably at least 16 % or 32 % of the circumference of the glass tube or of the glass preliminary product portion or of the glass product portion;
- at least one predetermined breaking plane with filaments extending obliquely relative to the local radius is provided between end-side fracture surfaces.

15. Use of glass tube portions or glass preliminary products or glass products according to any one of claims 12 to 14 for pharmaceutical packaging.

## Revendications

1. Procédé de fabrication de produits de départ en verre et de produits en verre, avec des étapes suivantes :
- de fourniture de tube en verre (2) ou de produit de départ en verre de forme tubulaire,
- d'exposition à base de laser du tube en verre (2) ou du produit de départ en verre à un rayonnement (30) focalisé pour produire des filaments (6) dans un plan de séparation (20) souhaité du tube en verre (2) ou du produit de départ en verre ;
- dans lequel l'exposition à un rayonnement (30) focalisé dans le plan de séparation (20) est effectuée pour produire les filaments (6) selon un angle oblique d'exposition (a) divergeant de la perpendiculaire par rapport à la surface locale du tube en verre (2) ou du produit de départ en verre, dans lequel les filaments s'étendent depuis l'extérieur vers l'intérieur.

2. Procédé selon la revendication 1,
dans lequel des tronçons (21) dimensionnés du tube en verre (2) ou du produit de départ en verre sont détachés par une tension (de traction) appliquée de manière mécanique ou thermique dans le plan de séparation (20) du tube en verre (2) ou du produit de départ en verre et une face de rupture est produite le long du plan de séparation (20).

3. Procédé selon la revendication 1 ou 2,
dans lequel le tube en verre (2) ou le produit de départ en verre et le système d'exposition à un rayonnement (30) focalisé tournent relativement l'un par rapport à l'autre de telle sorte que le rayonnement (30) focalisé rencontre le tube en verre (2) ou le produit de départ en verre dans le plan de séparation (20) souhaité.

4. Procédé selon l'une quelconque des revendications 1 à 3, avec au moins une des caractéristiques suivantes :
- l'angle oblique d'exposition (a) se situe dans la plage de 89,5° à 75°, de manière préférée dans la plage entre 85° et 80°
- un décalage (d) du rayonnement (30) focalisé, par rapport à la direction localement radiale, mesuré sur la surface du tube en verre ou du produit de départ en verre, dans la plage de 0,1 mm à 3 mm est effectué pour le tube en verre (2) ou pour le produit de départ tubulaire en verre dans la plage de diamètres de 3 mm à 50 mm suite à l'angle oblique de rayonnement a.

5. Dispositif de fabrication de produits de départ en verre de forme tubulaire ou de produits en verre, comprenant :
- un système d'amenée (1) de tube en verre (2) ou de produit de départ en verre,
- un système d'exposition à un rayonnement (3) à base de laser pour produire un rayonnement (30) focalisé le long d'un plan de séparation (20) souhaité et selon un angle oblique d'exposition (a) par rapport à la surface locale du tube en verre (2) ou du produit de départ en verre au moyen d'une optique de focalisation (31),
- un système de guidage (4) pour guider l'optique de focalisation (31) le long d'un plan de séparation (20) souhaité à la distance souhaitée et selon l'angle oblique d'exposition (a) souhaité par rapport à la surface du tube en verre ou du produit de départ en verre pour produire des filaments, dans lequel les filaments s'étendent depuis l'extérieur vers l'intérieur, ainsi
- qu'un système d'évacuation (5) pour des tronçons de tube en verre (21) ou des produits de départ en verre dimensionnés.

6. Dispositif selon la revendication 5, **caractérisé par** au moins une des caractéristiques suivantes :
- le système de guidage (4) est réalisé pour faire circuler en orbite le système d'exposition (3) à base de laser autour du tube en verre (2) ou du produit de départ en verre,
- le système d'exposition (3) à base de laser et le système de guidage (4) sont réalisés de manière entraînée conjointement par rapport à la direction d'amenée de tube en verre (2) ou de produit de départ en verre,
- le système d'amenée (1) fait partie d'un système de production ou d'un système de façonnage pour tube en verre ou produit de départ en verre.

7. Dispositif selon l'une quelconque des revendications 5 ou 6, avec au moins une des caractéristiques suivantes :
- le système d'exposition (3) à base de laser contient un laser (32), une optique de focalisation (31) et une fibre en verre creux (34) les reliant et dans lequel le système de guidage (4) fait partie d'un robot, qui rend actif l'optique de focalisation (31) à une distance souhaitée et selon une orientation angulaire souhaitée par rapport à la surface du tube en verre (2) ou du produit de départ en verre dans un plan de séparation souhaité tout autour du tube en verre (2) ou du produit de départ en verre,
- l'optique de focalisation (31) est contenue dans une tête de guidage (33), qui présente un capteur de distance (35) pour définir la distance par rapport à la surface du tube en verre (2) ou du produit de départ en verre et un capteur de balayage (36) pour définir la position de filaments (6) produits,
- le système d'exposition (3) à base de laser présente une tête de scanner (41) rotative avec guidage de rayon par rapport à l'optique de focalisation (31), qui est disposée au niveau du côté intérieur d'un miroir (42), qui forme un espace annulaire, à travers lequel s'étend le verre de tube (2) ou le produit de départ en verre à pourvoir de filaments (6).

8. Dispositif selon la revendication précédente, avec au moins une des caractéristiques supplémentaires :
- le miroir présente des tronçons (43) disposés le long d'une vis,
- des tronçons du miroir sont réalisés en tant qu'optique de reproduction (44),
- plusieurs têtes de scanner rotatives sont disposées autour du tube en verre à usiner.

9. Produit de départ en verre ou produit en verre de forme tubulaire, pouvant être fabriqué selon l'une quelconque des revendications 1 à 4, sous forme de verre tubulaire (2),
dans lequel sont appliqués dans le plan de séparation (20) souhaité des filaments (6), qui s'étendent de manière oblique par rapport aux rayons de paroi respectivement locaux et depuis l'extérieur vers l'intérieur.

10. Produit de départ en verre ou produit en verre de forme tubulaire, pouvant être fabriqué selon l'une quelconque des revendications 1 à 4, sous la forme d'ampoules, de cartouches ou de corps de seringue, dont les corps sont façonnés à partir de verre tubulaire,
dans lequel sont appliqués, dans le plan de séparation (20) souhaité, des filaments (6), qui s'étendent de manière oblique par rapport aux rayons de paroi respectivement locaux et depuis l'extérieur vers l'intérieur.

11. Produit de départ en verre ou produit en verre de forme tubulaire selon la revendication 10, dont les corps sont pourvus au niveau du plan de séparation (20) souhaité respectivement d'un point de rupture théorique aux fins du détachement ultérieur.

12. Tronçon de tube en verre ou tronçon de produit de départ en verre, fabriqué avec le procédé selon l'une quelconque des revendications 2 à 4,
dans lequel une face de rupture produite affiche au niveau de l'extrémité de tronçon des filaments (6) cassés s'étendant de manière oblique par rapport aux rayons de paroi respectivement locaux.

13. Tronçon de tube en verre ou tronçon de produit de départ en verre ou tronçon de produit en verre selon la revendication 12, **caractérisé par** au moins une des caractéristiques suivantes :
- les filaments (6) adoptent une distance (61) les uns des autres dans la plage de 2 µm à 15 µm, de manière préférée dans la plage de 3 µm à 12 µm et de manière particulièrement préférée dans la plage de 4 µm à 8 µm,
- les filaments (6) font partie de deux ou plusieurs zones de filaments (60), qui sont disposées de manière répartie autour de la périphérie du tube en verre ou du tronçon de produit de départ en verre ou du tronçon de produit en verre et sont séparées les unes des autres par des zones non concernées, et dans lequel les zones de filaments (60) contiennent respectivement un groupe de filaments individuels, qui eux-mêmes sont tenus à distance les uns des autres, dans lequel les zones non concernées présentent entre les zones de filaments (60) de préférence une étendue mesurée dans la direction périphérique du produit d'au moins 50 µm ou de préférence d'au moins 100 µm.

14. Tronçon de tube en verre ou tronçon de produit de départ en verre ou tronçon de produit en verre selon la revendication précédente, avec au moins une des caractéristiques suivantes :
- jusqu'à douze zones de filaments (60) sont réparties autour de la périphérie du tube en verre ou du tronçon de produit de départ en verre ou du tronçon de produit en verre,
- les zones de filaments (60) adoptent au moins 8 %, de manière préférée au moins 16 % ou 32 % de la périphérie du tube en verre, ou du tronçon de produit de départ en verre ou du tronçon de produit en verre,
- au moins un plan de rupture théorique avec des filaments s'étendant de manière oblique par rapport aux rayons locaux est présent entre des faces de rupture côté extrémité.

15. Utilisation de tronçons de tube en verre ou de produits de départ en verre ou de produits en verre selon l'une quelconque des revendications 12 à 14 pour des emballages pharmaceutiques.
